# EUROPEAN PATENT APPLICATION

(11) **EP 3 957 308 A1**
(43) Date of publication of application: **23.02.2022**
(21) Application number: 20382764.7
(22) Date of filing: 21.08.2020
(51) Int. Cl.: A61K 31/426, A61P 11/00, A61P 11/06

(54) **(1R,3S)-3-((5-CYANO-4-PHENYLTHIAZOL-2-YL)CARBAMOYL)CYCLOPENTANE-1-CARBOXYLIC ACID AND DERIVATIVES THEREOF FOR USE IN THE TREATMENT OF AIRWAY DISEASES**

(71) Applicant: Palobiofarma, S.L., 08302 Mataró-Barcelona (ES)
(72) Inventor: CASTRO-PALOMINO LARIA, Julio, 08302 Mataró, Barcelona (ES); CAMACHO GÓMEZ, Juan, 31001 Pamplona, Navarra (ES); CASTRO-PALOMINO LARIA, Nahomi, 08302 Mataró, Barcelona (ES); ARIOSA ÁLVAREZ, Alina, 08013 Barcelona (ES)
(74) Representative: ABG Intellectual Property Law, S.L.

(57) **Abstract**

The present invention relates to (1R,3S)-3-((5-cyano-4-phenylthiazol-2-yl)carbamoyl)cyclopentane-1-carboxylic acid, its pharmaceutically acceptable salts and co-crystals thereof and to pharmaceutical compositions comprising said compound for use in the treatment of airway diseases, such as allergic asthma, chronic obstructive pulmonary disease (COPD), idiopathic pulmonary fibrosis (IPF), obstructive sleep apnea, allergic rhinitis, among others, in subjects having elevated levels of eosinophils in peripheral blood.

## Description

### FIELD OF THE INVENTION

The present invention relates to (1 R,3S)-3-((5-cyano-4-phenylthiazol-2-yl)carbamoyl)cyclopentane-1-carboxylic acid, its pharmaceutically acceptable salts and co-crystals thereof and to pharmaceutical compositions comprising said compound for use in the treatment of airway diseases, such as allergic asthma, chronic obstructive pulmonary disease (COPD), idiopathic pulmonary fibrosis (IPF), obstructive sleep apnea, allergic rhinitis, among others in subjects having elevated levels of eosinophils in peripheral blood.

Other objectives of the present invention are to provide methods for the treatment of diseases by administration to subjects having elevated levels of eosinophils in peripheral blood of compound of formula (I), or by administration of a pharmaceutical composition or a combination product comprising compound of formula (I).

### STATE OF THE ART

Eosinophilia is a condition which is sometimes associated with airway diseases such as asthma, COPD, allergic rhinitis, among others. Many of these diseases are treated with corticosteroids (topical or systemic), which target both eosinophils and other immune cells such as lymphocytes that might be the inciting cell population. (Kuang, F. L, Approach to Patients with Eosinophilia, Med Clin N Am 104 (2020) 1-14).

Specifically, blood eosinophils have been extensively described as a surrogate biomarker of airway inflammation, a feature of asthma and COPD phenotypes in specific subjects. The measurement of contemporaneous eosinophilia in peripheral blood cells has been investigated in recent years as a potential surrogate marker of bronchial and/or lung inflammation. In most of the studies, a cut-off blood eosinophils value of 300 cel/uL is considered to classify asthmatic patients to have eosinophilic asthma. (Kostikas, K et al, Blood Eosinophils as Biomarkers to Drive Treatment Choices in Asthma and COPD, Curr Drug Targets. 2018 Dec; 19(16): 1882-1896).

Currently, eosinophil-targeted therapy has been shown to reduce disease flares, some of them achieving governmental approval in severe eosinophilic asthma and eosinophilic granulomatosis with polyangiitis. The above therapies are associated with the use of biologics products, like monoclonal antibodies. Few small molecules drugs are being evaluated in a variety of eosinophil-associated conditions. (Klion, A. D et al, Contributions of Eosinophils to Human Health and Disease, Annu. Rev. Pathol. Mech. Dis. 2020.15:179-209).

Although the differential for eosinophilia is broad, eosinophilia secondary to atopy is common due to the frequency of atopy in the population at large. Tissue eosinophils are thought to contribute to end-organ fibrosis and damage in uncontrolled atopic inflammation in diseases like asthma and eosinophilic gastrointestinal disorders. Mild to moderate peripheral eosinophilia is a common finding seen in atopic diseases; however, eosinophils in atopic disorders typically do not infiltrate additional tissues beyond those primarily affected by the atopic disorder. There is growing evidence that the peripheral eosinophil count can potentially be used as a biomarker, which may correlate with disease activity in some atopic disorders. (Cafone, J et al, The Role of Eosinophils in Immunotherapy, Current Allergy and Asthma Reports (2020) 20:1).

### Asthma

Asthma is a heterogeneous disease, usually characterized by chronic airway inflammation. It is defined by the history of respiratory symptoms such as wheeze, shortness of breath, chest tightness and cough that vary over time and in intensity, together with variable expiratory airflow limitation. These variations are often triggered by factors such as allergen or irritant exposure, viral respiratory infections, exercise or change in weather.

One of the asthmatic clinical phenotypes identified and characterized is allergic asthma, which is felt to be largely mediated by the Th2 inflammatory pathway and is associated with high specific IgE levels. Airway eosinophilia is a common feature of allergic asthma, and therefore, allergic asthmatics usually share the phenotype with the pathogenesis of eosinophilic asthma. (Global Strategy for Asthma Management and Prevention. 2020. Available from: www.ginasthma.org).

An alternative treatment for allergic asthma is based on specific immunotherapy, that is, the repeated administration of increasing doses of allergens to induce hyposensitivity and, therefore, reduce symptoms when another subsequent exposure to said allergen occurs. The efficacy of immunotherapy is known to remain limited and highly variable among patients.

Another alternative of treatment for allergic asthma is related to the use of biologics, such as monoclonal antibodies, which are given parenterally / intravenously. The antibodies themselves are proteins, so giving them can sometimes cause something like an allergic reaction, or other side effects related to the antigens they target. Additionally, they can represent high costs, complex dosing and management, and are not widely available around the world.

Theophylline, a small molecule xanthine-derivative, has been used in the treatment of asthma for more than 60 years, showing significant side effects at doses that are needed for bronchodilation. Theophylline is a weak, non-selective adenosine antagonist and, in addition, an inhibitor of different families of phosphodiesterases. Therefore, it shows a low therapeutic index and drug levels must be closely monitored. (Scheiff A. B. et al, 2-Amino-5-benzoyl-4-phenylthiazoles: Development of potent and selective adenosine A1 receptor antagonists, Bioorg. Med. Chem. 18 (2010) 2195-2203).

Several compounds adenosine A1 receptor antagonists, which are selective and non-xanthine derivatives, have reached stages of clinical development in humans, related to indications of heart failure and renal dysfunction, but none of them have confirmed efficacy of said treatments. Other compounds of the same class were proposed for other indications such as liver fibrosis / steatosis, sepsis, neurodegenerative and movement disorders (compound with dual activity antagonism A_{2A} / A₁) but did not reach the stages of clinical development. (Giorg I et al, Adenosine A1 modulators: a patent update (2008 to present*),* Expert Opin. Ther. Patents (2013) 23(9)).

### COPD

There are others chronic lung diseases which may be associated in some patients with eosinophilic airway inflammation, for example, chronic obstructive pulmonary disease (COPD). Two observational studies have showed that the blood eosinophil count is the stand-out biomarker of an exacerbation of COPD associated with a raised sputum eosinophil count and a positive response to prednisolone. (Pavord, I. D., Blood Eosinophil-directed Management of Airway Disease: The Past, Present and Future, AJRCCM Articles in Press. Published May 01, 2020).

In chronic obstructive pulmonary disease (COPD) the international diagnostic and treatment guidelines do not incorporate recommendation for the treatment of allergy. This lack of recommendations is largely due to insufficient knowledge on the role of atopy in the pathogenesis and outcome of COPD. Nevertheless, it has been reported that around 18% of COPD patients are atopic and that atopy is a possible risk factor for developing COPD. Therefore, there has been a growing interest in finding the link between atopy and COPD and its consequence on the disease outcome.

European Respiratory Society on Chronic Obstructive Pulmonary Disease (EUROSCOP) has conducted a large multi-centre study to assess the effect of 3-year treatment with inhaled budesonide on lung function decline in smoking COPD patients, and concluded that atopy is present in COPD patients and it is associated with a higher prevalence and incidence of respiratory symptoms. The study remark that the atopic status should not be forgotten in the routine work-up of COPD. (Fattahi, F. et al, Atopy is a risk factor for respiratory symptoms in COPD patients: results from the EUROSCOP study, Respiratory Research 2013, 14:10).

### IPF

Idiopathic pulmonary fibrosis (IPF) encompasses a group of interstitial lung diseases, and it is the cause of death of an important number of people worldwide. There are only a few published studies on the association of sputum eosinophilia and idiopathic lung fibrosis. There are studies showing that the average percentage of sputum eosinophils was higher in IPF patients (2.1%) compared with normal individuals (0.3%; p < 0.001), but similar to levels in COPD patients. Besides, eosinophilic cationic protein was found to be higher in IPF (1.1 mg/ml) than in normal subjects (0.2 mg/ml) and even higher than COPD (0.4 mg/ml), which suggests that the inflammation is active and gives a possible relationship between eosinophilic inflammation in IPF and cough. Other study has reported that eosinophils are important in progressive IPF and are correlated with unfavorable outcome. (Eltboli O. et al, Eosinophils as diagnostic tools in chronic lung disease, Expert Rev. Respir. Med. 7(1), (2013)).

### Allergic rhinitis

Allergic rhinitis may present with a mild peripheral eosinophilia. Peripheral eosinophilia outperformed total IgE levels in predicting mucosal disease in this cohort, with a positive predictive value of 89% and negative predictive value of 99%. (Cafone, J et al, The Role of Eosinophils in Immunotherapy, Current Allergy and Asthma Reports (2020) 20:1).

### Obstructive sleep apnea

Obstructive sleep apnea (OSA) refers to repeated episodes of halted respiration during sleep, despite a continuous effort to breathe. Clinically, OSA is characterized by excessive daytime sleepiness, disruptive snoring, and nocturnal hypoxemia. This condition is a common sleep-breathing disorder affecting approximately 4.0% of males and 2.0% of females of middle age in the developed world. Many studies have shown that OSA is a significant source of morbidity and mortality; it is associated with serious health consequences, mostly afflicting the cardiovascular and cerebrovascular systems. There are studies demonstrating that allergic rhinitis related inflammation might worsen the severity of obstructive sleep apnea (OSA). (Gadi, G et al, The prevalence of allergic rhinitis and atopic markers in obstructive sleep apnea, Journal of Epidemiology and Global Health Volume 7, Issue 1, March 2017, Pages 37-44).

There is an unmet medical need to have an effective and safe treatment for airway diseases in subjects having elevated levels of eosinophils in peripheral blood, which guarantees a control of these diseases in said group of subjects and an improvement in the clinical conditions of said patients and its quality of life, all accompanied by simple administration procedures. For this, oral treatments and with the minimum daily dosages are considered a desirable alternative.

International patent application WO 2009/044250 A1 discloses a group of 5-cyanothiazol-2-yl acetamide derivatives as antagonists of the adenosine A₁ receptors and their use in the treatment of conditions or diseases susceptible of amelioration by antagonism of said adenosine receptor. Treatment of airway diseases in subjects having elevated level of eosinophils in peripheral blood is not specifically recited in WO 2009/044250 A1.

Currently, no selective adenosine A₁ receptor ligand has obtained market authorization. Furthermore, none of them have been tested in human clinical trials for the treatment of airway diseases, such as allergic asthma, chronic obstructive pulmonary disease (COPD), idiopathic pulmonary fibrosis (IPF), obstructive sleep apnea, allergic rhinitis, among others.

The inventors of the present patent application have studied the effectiveness of Compound (I) in the treatment of airway diseases, specifically in mild-to-moderate allergic asthmatics, and have unexpectedly found that subjects treated with Compound (I) for 15 days experiment a significant decrease in peripheral eosinophil counts and a significant increase in trough forced expiratory volume (trough FEV1) and in the corresponding FEV1 AUC_{30min-23h 30min,} together with an improvement in the Asthma Control Questionnaire-7 (ACQ-7) score. The improvement is particularly remarkable in patients having elevated level of eosinophils in peripheral blood, in particular a level of eosinophils in peripheral blood equal to or greater than 300 cel/µL.

Therefore, there is ongoing interest to develop oral once-daily medications to further simplify treatment regimens of airway diseases and improve patient compliance.

The inventors of the current invention have now surprisingly found that (1R,3S)-3-((5-cyano-4-phenylthiazol-2-yl)carbamoyl)cyclopentane-1-carboxylic acid of formula (I) 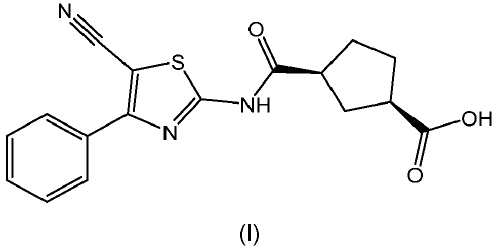 is particularly efficacious for the treatment of airway diseases producing a significant decrease in peripheral eosinophil counts in subjects having, before said treatment, an elevated level of eosinophils in peripheral blood, in particular a level of eosinophils in peripheral blood equal to or greater than 300 cel/µL.

In one aspect the present invention provides (1R,3S)-3-((5-cyano-4-phenylthiazol-2-yl)carbamoyl)cyclopentane-1-carboxylic acid of formula (I), its pharmaceutically acceptable salts and cocrystals thereof for use in the treatment of airway diseases in subjects having, before said treatment, a level of eosinophils in peripheral blood equal to or greater than 300 cel/µL. Compound of formula (I) has the ability to reduce blood eosinophil count. Another advantage is given by the excellent toxicity profile of said compound tested in humans, in comparison with other medicaments for the treatment of airway eosinophilic diseases as corticoids and biological products and others adenosine A₁ antagonists known in the state of the art. Another differential point is the possibility to be administered orally.

### SUMMARY OF THE INVENTION

The present invention relates to (1R,3S)-3-((5-cyano-4-phenylthiazol-2-yl)carbamoyl)cyclopentane-1-carboxylic acid, its pharmaceutically acceptable salts and co-crystals thereof, to pharmaceutical compositions comprising said compounds and to combinations of said compounds with one or more agent useful in the treatment of airway diseases, such as allergic asthma, chronic obstructive pulmonary disease (COPD), idiopathic pulmonary fibrosis (IPF), obstructive sleep apnea, allergic rhinitis, among others in subjects having, before said treatment, an elevated level of eosinophils in peripheral blood, in particular a level of eosinophils in peripheral blood equal to or greater than 300 cel/µL.

### DETAILED DESCRIPTION OF THE INVENTION

In one aspect the present invention relates to (1R,3S)-3-((5-cyano-4-phenylthiazol-2-yl)carbamoyl)cyclopentane-1-carboxylic acid of formula (I) (Compound (I)) 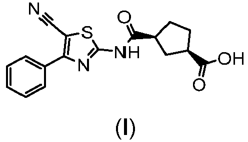 its pharmaceutically acceptable salts or co-crystals thereof for use in the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In another aspect the present invention relates to the use of (1R,3S)-3-((5-cyano-4-phenylthiazol-2-yl)carbamoyl)cyclopentane-1-carboxylic acid of formula (I) (Compound (I), its pharmaceutically acceptable salts or co-crystals thereof for the manufacture of a medicament for the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In yet another aspect the present invention relates to the use of a pharmaceutical composition comprising (1 R,3S)-3-((5-cyano-4-phenylthiazol-2-yl)carbamoyl) cyclopentane-1-carboxylic acid of formula (I), its pharmaceutically acceptable salt or co-crystals thereof for use in the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In still another aspect the present invention relates to a combination product comprising (1 R,3S)-3-((5-cyano-4-phenylthiazol-2-yl)carbamoyl)cyclopentane-1-carboxylic acid of formula (I), its pharmaceutically acceptable salts or co-crystals thereof and one or more agent useful in the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In yet another aspect the present invention relates to a combination as described in the previous paragraph for use in the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In yet another aspect of the present invention refers to methods for the treatment of airway diseases, such as allergic asthma, chronic obstructive pulmonary disease (COPD), idiopathic pulmonary fibrosis (IPF), obstructive sleep apnea, allergic rhinitis, by administration to subjects having, before said treatment, a level of eosinophils in peripheral blood equal to or greater than 300 cel/µL of:
- A compound of formula (I) or a pharmaceutically acceptable salts or co-crystals thereof, or
- A pharmaceutical composition comprising compound of formula (I) or
- pharmaceutically acceptable salts or co-crystals thereof, or
- A combination product comprising compound of formula (I) or a pharmaceutically acceptable salt or co-crystals thereof.

As it is said before, the compound of formula (I) of the invention is useful in the treatment of airway diseases, such as allergic asthma, chronic obstructive pulmonary disease (COPD), idiopathic pulmonary fibrosis (IPF), obstructive sleep apnea, allergic rhinitis.

Accordingly, the compound of formula (I) of the present invention and pharmaceutically acceptable salts or co-crystals thereof, pharmaceutical compositions comprising such compound and / or salts or co-crystals thereof, and combination products comprising such compound, salts or co-crystals thereof, may be used in a method of treatment airway diseases, such as allergic asthma, chronic obstructive pulmonary disease (COPD), idiopathic pulmonary fibrosis (IPF), obstructive sleep apnea, allergic rhinitis, which comprises administering to a subject having, before said treatment, a level of eosinophils in peripheral blood equal to or greater than 300 cel/µL in need of said treatment, an effective amount of the compound of formula (I) of the invention or a pharmaceutically acceptable salt or co-crystal thereof.

In a preferred embodiment (1R,3S)-3-((5-cyano-4-phenylthiazol-2-yl)carbamoyl) cyclopentane-1-carboxylic acid of formula (I), its pharmaceutically acceptable salt or co-crystals thereof, the combinations comprising said compounds and one or more agent useful in the treatment of airway diseases, such as allergic asthma, chronic obstructive pulmonary disease (COPD), idiopathic pulmonary fibrosis (IPF), obstructive sleep apnea, allergic rhinitis in subjects having, before said treatment, a level of eosinophils in peripheral blood equal to or greater than 300 cel/µL.

In a preferred embodiment (1R,3S)-3-((5-cyano-4-phenylthiazol-2-yl)carbamoyl) cyclopentane-1-carboxylic acid of formula (I) is administered to the subject having, before said treatment, a level of eosinophils in peripheral blood equal to or greater than 300 cel/µL in a therapeutically effective amount, for the treatment of airway diseases, such as allergic asthma, chronic obstructive pulmonary disease (COPD), idiopathic pulmonary fibrosis (IPF), obstructive sleep apnea, allergic rhinitis, wherein the levels of blood eosinophils is reduced following administration.

Some embodiments are directed to methods of treating a condition characterized by elevated levels of peripheral eosinophils in a subject comprising administering to the subject in need thereof a therapeutically effective amount of (1R,3S)-3-((5-cyano-4-phenylthiazol-2-yl)carbamoyl)cyclopentane-1-carboxylic acid of formula (I) or a pharmaceutically acceptable salt thereof, wherein the level of blood eosinophils is reduced.

In a preferred embodiment (1R,3S)-3-((5-cyano-4-phenylthiazol-2-yl)carbamoyl) cyclopentane-1-carboxylic acid of formula (I) is administered for the treatment of a condition characterized by elevated blood eosinophils levels. In a more preferred embodiments, eosinophilic disease is characterized by elevated levels of eosinophils in the peripheral blood equal to or greater than 300 cell/µL.

In an embodiment, the method of the present invention provides a therapeutic effect such that the patient achieves a reduction in peripheral eosinophil counts between 5% - 30% within 4 weeks, preferably within 2 weeks of beginning the treatment.

In a preferred embodiment, the airway disease is asthma selected from atopic asthma, allergic asthma, mild asthma, moderate asthma, severe asthma, eosinophilic asthma and combinations thereof. In a preferred embodiment, the compound of formula (I) or pharmaceutically acceptable salt or co-crystals thereof for use in the treatment of asthma wherein the allergic asthma is selected from mild, moderate and severe asthma.

In a preferred embodiment, the condition is COPD.

In a preferred embodiment, the condition is IPF.

In a preferred embodiment, the compound of formula (I) or pharmaceutically acceptable salt or co-crystals thereof for use in the treatment of airway diseases is for administration by oral route.

In a preferred embodiment, the compound of formula (I) or pharmaceutically acceptable salt or co-crystals thereof for use in the treatment of allergic asthma is administered once or twice a day. In a more preferred embodiment, the compound of formula (I) is administered once a day.

In a preferred embodiment, the compound of formula (I) or pharmaceutically acceptable salt or co-crystals thereof for use in the treatment of airway diseases is administered at a dose between 5 mg - 40 mg. In a more preferred embodiment, the compound of formula (I) is administered at a dose between 5 mg - 20 mg.

In a preferred embodiment, pharmaceutical composition comprises an effective amount of compound of formula (I) or a pharmaceutically acceptable salt or co-crystal thereof and a pharmaceutically acceptable vehicle or carrier.

In a preferred embodiment, a combination product comprises a compound of formula (I) or a pharmaceutically acceptable salt or co-crystal thereof, and one or more agent selected from the group consisting of corticosteroids, such as budesonide, fluticasone, beclomethasone, mometasone, bronchodilators such as salmetherol and formoterol, and biologic products selected from Dupilumab, Reslizumab, Mepolizumab, Imatinib, Lebrikizumab, AK002, Benralizumab, Tralokinumab, and anti-fibrotic drugs such as Pirfenidone and Nintedanib. The components of the combination product are in the same formulation or in separate formulations.

In one embodiment of the present invention the compound of formula (I), its pharmaceutically acceptable salts or its co-crystals and the agents useful in the treatment of allergic asthma form part of the same composition.

In another embodiment of the present invention the compound of formula (I), its pharmaceutically acceptable salts or its co-crystals and the agents useful in the treatment of allergic asthma form part of separate compositions for administration simultaneously or sequentially.

In a preferred embodiment, the method of treatment of allergic asthma has an effect which can be measured by any suitable metric, selected from peripheral eosinophil counts and trough forced exhale volume in 1 second (FEV1). Methods of measuring of peripheral eosinophil counts and trough FEV1 and are known in the art. These and other methods of determining the effect of treatment on a patient can be used alone or in combination.

Eosinophil count. Peripheral blood eosinophil counts were obtained from standard complete blood counts done at every centre participating in the study. For example, by using standardized methods on a Beckman Coulter LH series analyzer (Beckman Coulter Ltd, Brea, CA, USA), Negewo, N. A. et al, Peripheral blood eosinophils: a surrogate marker for airway eosinophilia in stable COPD, Int J Chron Obstruct Pulmon Dis. 2016; 11: 1495-1504).

Eosinophils in S.I units (10⁹/L) will be converted in cel/µL with the conversion factor 1 x 10⁹/L =1000 cel/µL. The primary efficacy analysis on Per Protocol Population was stratified into 2 subgroups based on Eosinophils (cel/ µL) value at visit V1 from Hematology (Low < 300 cel/ µL, and High >=300 cel/ µL). Trough FEV1 on day 16 (+4-day leeway) was summarized by treatment arm and Eosinophils value at visit V1. A mixed model with FEV1 measurements on day 16 (23h 15min and 23h 45min post-dose), containing treatment arm and Eosinophils strata as fixed effects, site as random effect with baseline FEV1 on visit V5 as a covariate was fitted.

FEV1. Briefly. Spirometry measurements will be performed using spirometry equipment which meets or exceeds the minimal performance recommendations of the ATS/ERS standards (Miller M. R et al, Standardisation of spirometry, Eur Respir J 2005; 26: 319-338). Calibration and quality control should be performed before the site initiation:
a. A 3L syringe will be used to verify spirometer accuracy.
b. The accuracy of the syringe should be certified within the last 12 months and should have an accuracy of ± 15 ml.
c. Before use, the syringe should be maintained at the same temperature as the spirometer to ensure volume stability.
d. For volume-type spirometers, a calibration and leak check must be performed before each testing session according to the manufacturer's guidelines. A calibration report stored in the site's source documents is required to confirm the adherence to guidelines. Example of spirometry equipment used in the trial: Jaeger and ERT Spirosphere.

The present invention may be employed preferably a mammal, more preferably a human subject.

### DESCRIPTION OF THE FIGURES

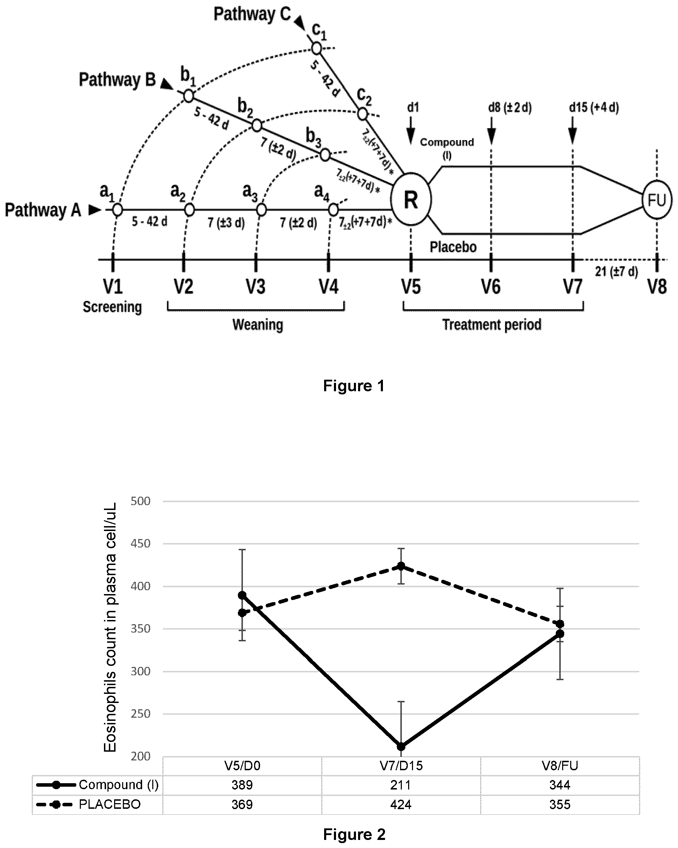

**Figure 1**. Study design. The study comprises: a screening visit (V1); a weaning protocol for asthma maintenance therapy (visits V2 to V4); a two-arm treatment period (visits V5 to V7, where V5 is the randomization visit, R); and a follow-up visit (V8, FU). Subjects enter screening and undergo the asthma treatment weaning period through 3 alternative pathways (A, B or C) depending on their asthma medication at inclusion in the study. a1, b1 and c1: visit V1 in pathways A, B, and C, respectively. Subjects on medium-dose ICS plus at least one more asthma controller medicine undergo the weaning period through Pathway A with the following steps: (a2) withdrawal of non-ICS medication (LABA, LTRA) on visit V2; (a3) ICS reduction to low-dose on visit V3; and (a4) low-dose ICS withdrawal on visit V4. Subjects on low-dose ICS plus at least one more asthma controller, or medium-dose ICS as asthma monotherapy, undergo Pathway B as follows: (b2) withdrawal of non-ICS medication (LABA, LTRA), or reduction of ICS monotherapy from medium to low dose as applicable; and (b3) withdrawal of low-dose ICS. These subjects skip visit V4 and go directly from V3 (step b3) to randomization. Subjects who enter the study on low-dose ICS monotherapy undergo Pathway C, where their ICS is withdrawn on visit V2 (single step c2) and proceed to randomization skipping visits V3 and V4. Rhinitis/rhinosinusitis intranasal medication is discontinued on visit V2 for all subjects where applicable. Time intervals before randomization (R) refer to distances between visits. For visits V5 (randomization), V6 and V7, the exact visit day and its leeway are plotted (*Visit V5 latency: visit V5 must first be appointed at 7 days (± 2 days) after visit V4 for Pathway A, visit V3 for Pathway B or visit V2 for Pathway C. For those subjects who do not meet the spirometric inclusion criterion No.10, the visit V5 procedures can be interrupted, and subsequent unscheduled visits can be appointed in a maximum of 7 days for spirometric follow-up. A full visit V5 can be rescheduled up to a maximum of 14 days later after the first V5 interruption with a minimum of 1 follow up visit in between). The time interval represented from visit V7 to V8 (FU, follow-up visit) is also applicable to a PSW (premature subject withdrawal) visit after the withdrawal date. PATHWAY A: Subjects who enter the study on medium-dose ICS plus at least one more asthma controller medicine (e.g. LABA or LTRA). All weaning visits: V1 → V4. PATHWAY B: Subjects who enter the study on low-dose ICS plus at least one more asthma controller medicine, or medium-dose ICS as asthma monotherapy. Weaning visits V1 → V3 (no V4). PATHWAY C: Subjects who enter the study on low-dose ICS as asthma monotherapy. ICS withdrawn on visit V2 (no V3 nor V4). Rescue medication: Short-acting β2-adrenergic rescue bronchodilator available to the subjects throughout the entire study.
**Figure 2**. Effect of Compound (I) administration on eosinophils count. V5/D0: Visit 5, day 0. V7/D15: Visit 7, day 15. V8/FU: Visit 8, Follow up.
**Figure 3****.** Differences in FEV1 regarding baseline after 15 days of Compound (I) / Placebo treatment (serial spirometries).
**Figure 4****.** AUC of FEV1 after 15 days of administration.
**Figure 5**. Decrease in ACQ-7 score regarding baseline after Compound (I) / Placebo treatment.
**Figure 6**. Summary of related adverse events by system organ class and preferred term worst grade per patient (Safety Population).

As used in the present document the term allergic asthma is used to designate a type of asthma that causes symptoms when a person is around certain triggers (allergens). These allergens lead to an immune system response that affects the lungs and makes it harder to breathe. (https://www.medicalnewstoday.com/articles/324476).

As used in the present document the term atopic asthma, like allergic asthma, is typically associated with heightened immune responses to inhaled allergens.

As used in the present document the term *mild asthma* is used to designate asthma that is well controlled with treatment with regular daily low dose ICS, with as-need SABA, being effective in reducing asthma symptoms and reducing the risk of asthma-related exacerbations, hospitalization and death, but adherence with ICS is poor. Also, in adults and adolescents with mild asthma, treatment with as-need low dose ICS-formoterol reduces the risk of severe exacerbations by about two-thirds compared with SABA-only treatment and is non-inferior to daily low dose ICS for severe exacerbations. *(*Global Strategy for Asthma Management and Prevention. 2020. Available from: www.ginasthma.org).

As used in the present document the term *moderate asthma* is used to designate asthma that is controlled with treatment with combination low dose ICS-LABA as maintenance treatment with as-needed SABA as reliever, and low dose ICS-formoterol as both maintenance and reliever treatment. Also, another treatment scheme for moderate asthma include medium dose ICS plus as-needed SABA or low combination ICS-LABA plus as-needed SABA. (Global Strategy for Asthma Management and Prevention. 2020. Available from: www.ginasthma.org).

As used in the present document the term *severe asthma* is used to designate asthma that is uncontrolled despite GINA Step 4 or 5 treatment or that requires such treatment to maintain good symptom control and reduce exacerbations. Approximately 3-10 % of people with asthma have severe asthma. (Global Strategy for Asthma Management and Prevention. 2020. Available from: www.ginasthma.org).

As used herein, the term pharmaceutically acceptable salt is used to designate salts with a pharmaceutically acceptable base such as alkali metal (e.g. sodium or potassium), alkali earth metal (e.g. calcium or magnesium) hydroxides, and organic bases, for example alkyl amines, arylalkyl amines and heterocyclic amines.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and ptoluenesulphonate. X- is preferably an anion selected from chloride, bromide, iodide, sulphate, nitrate, acetate, maleate, oxalate, succinate or trifluoroacetate.

As used herein, the term co-crystals is used to designate crystalline materials composed of two or more molecules in the same crystal lattice, more particularly co-crystals formed by a molecule of (1R,3S)-3-((5-cyano-4-phenylthiazol-2-yl)carbamoyl)cyclopentane-1-carboxylic acid of formula (I), and a pharmaceutically acceptable coformer.

### Example

### Example 1. Study to investigate the effect of the adenosine A₁ receptor antagonist (1R,3S)-3-((5-cyano-4-phenylthiazol-2-yl)carbamoyl)cyclopentane-1-carboxylic acid on forced expiratory volume in 1 second (FEV1) in patients with mild-to-moderate persistent atopic asthma

### Aim

The present trial is an exploratory study aiming at evaluating the safety, tolerability, and efficacy of a 15-day, once daily administration of 10 mg Compound (I) in subjects with persistent mild-to-moderate atopic asthma. In terms of exploratory efficacy, the primary purpose of this study is to determine whether Compound (I) compared to placebo, improves the FEV1, as well as to provide comparative safety data from this population of asthmatics. Measurements made in this study will also be used to establish whether this treatment improves other variables related to asthma control and lung function.

### Inclusion criteria

1. Written informed consent must be obtained before any study assessments are performed. Subjects must be able to communicate well with the investigator and staff so that they can understand and comply with the requirements of the study.
2. Male and female subjects of 18- 70 years age.
3. Subjects with a medical history of mild-to-moderate persistent allergic asthma, diagnosed according to GINA 2017 guidelines, and managed in therapeutic steps 2-3 being ICS limited to low/medium dose, or step 4 restricted to medium-dose ICS plus LABA and/or a leukotriene antagonist, as maintenance therapy. Asthma maintenance therapy must have been stable for at least three months before inclusion in the study. Subjects treated with intranasal medication for allergic rhinitis or chronic rhinosinusitis with or without nasal polyposis are allowed to enter the study. The use of oral antihistamines as rescue medication for allergic rhinitis is permitted, subjected to protocol-established washout periods.
4. A positive skin prick test or specific serum IgE to aeroallergens, such as house dust mite, tree or grass pollen, pet dander, or cockroach antigens. In addition, any allergens specific to the country/locality can be included. A historical skin prick test or serum IgE results within 12 months before screening is acceptable (supported with source documentation), except for subjects who have received specific immunotherapy within the past 12 months (see exclusion criterion No. 18). For such subjects, a positive aeroallergen skin prick test or specific serum IgE must be obtained on visit V1.
5. Women of child-bearing potential must agree to employ effective contraception from Visit 1 through FU visit, unless they are surgically sterile (i.e. bilateral tubal ligation, bilateral oophorectomy, or complete hysterectomy), are at least 2 years postmenopausal, or practice abstinence. Acceptable contraception procedures are oral, transdermal, or implanted contraceptives, intrauterine device, female condom with spermicide, diaphragm with spermicide, or use of a condom with spermicide by the sexual partner. Female subjects repotting surgical sterilization must have had the procedure done at least 6 months before the initial dosing of study medication. Surgical sterilization procedures must be supported with clinical documentation made available to the study sponsor.
6. All female subjects must have negative pregnancy test results at screening and baseline.
7. Male subjects must agree to use two acceptable methods of contraception, (e.g. spermicidal gel plus condom) for the entire duration of the study and up to the study completion visit, and refrain from fathering a child within the three months following the last study drug administration. Periodic abstinence and withdrawal are not acceptable methods of contraception.
8. Subjects must weigh at least 45 kg and must have a body mass index (BMI) ≥ 17 kg/m².
9. Subjects must demonstrate an increase of ≥12% AND ≥200 mL in FEV1 over their prebronchodilator value within 30 min after inhaling a total of 400µg of salbutamol (reversibility test). Reversibility can be determined at screening or during the weaning period up to visit V5. Alternatively, subjects who has not shown a positive "reversibility test" but have had a prebronchodilator FEV1 decrease ≥200 mL from their basal FEV1 on Visit V1 are equally eligible.
10. On Visit V5, upon completion of the weaning of asthma maintenance therapy, subjects must have a pre-bronchodilator FEV1 ≥60% and ≤90% of their predicted normal value, upon completion of LABA and ICS weaning. on Visit V5. Alternatively, subjects showing a pre-bronchodilator FEV1 ≥90% on Visit V5 must have had a pre-bronchodilator FEV1 decrease ≥200 mL from their basal FEV1 on Visit V1.
11. Subjects must have an ACQ-7 score ≥1.5 upon completion of LABA and ICS weaning on Visit V5.
12. Subjects must meet a ≥80% compliance with the morning and evening electronic/PEF meter recordings during the
13. Weaning of their asthma maintenance therapy (i.e. from visit V2 to visit V5).

### Exclusion criteria

1. Use of other investigational drugs at the time of enrollment, or within 30 days or 5 half-lives of enrollment, whichever is longer.
2. History of hypersensitivity to the study medication or drugs of similar chemical classes (A1 adenosine receptor antagonists).
3. A history of clinically significant ECG abnormalities or a recent history of autonomic dysfunction (e.g. recurrent episodes of fainting, arrhythmia, etc.).
4. History of malignancy of any organ system (other than localized basal cell carcinoma of the skin), treated or untreated, within the past 5 years.
5. Pregnant or nursing (lactating) women.
6. Smokers, defined by smoking within the previous 6 months or having a smoking history of more than 10 packs-years, a pack-year being defined as smoking the equivalent of 20 cigarettes (a pack) per day for 1 year.
7. Subjects with severe persistent asthma managed in GINA therapeutic step 4 (except for the restricted allowance in inclusion criterion 3) or 5 according to GINA 2017 guidelines. This criterion includes subjects treated with high-dose ICS, systemic corticosteroids, tiotropium bromide, theophylline or monoclonal antibody-based biological therapies such as omalizumab, mepolizumab, reslizumab, etc. Subjects treated with any immunosuppressant drug, or with systemic corticosteroids for any condition other than asthma, are excluded. Subjects requiring daily use of antihistamine drugs are also excluded.
8. Present or past use of a biologic (e.g. monoclonal antibodies) agent for the treatment of asthma. Use of a biologic agent for any other condition within the past 6 months.
9. Use of systemic corticosteroids to treat an asthma exacerbation or any other condition within 4 weeks prior to Visit V1.
10. History of life-threatening asthma, defined as an asthma episode that required intubation and/or was associated with hypercapnia, respiratory arrest and/or hypoxic seizures. History of asthma exacerbations that required ward hospitalization or an emergency room stay greater than 48 hours within 5 years prior to Visit 1.
11. Any disease or illness other than asthma that may require the use of systemic corticosteroids during the study period.
12. Any occupational exposure to allergens/irritants that may have a potential to worsen the asthma symptoms during the trial.
13. A respiratory tract infection requiring the use of antibiotics within 4 weeks prior to Visit V1, or pneumonia within 6 months prior to Visit V1.
14. An asthma exacerbation requiring treatment, or the use of any health care resources within 4 weeks prior to visit V1. This includes asthma exacerbations managed with a transient increase of the subject's regular asthma maintenance therapy, and self-managed exacerbations using an "action plan".
15. Subjects with any other underlying diseases that may compromise safety or may interfere with efficacy outcomes (e.g. tuberculosis, clinically relevant bronchiectasis, diffuse lung interstitial disease, pulmonary hypertension, emphysema, chronic bronchitis, α-1-antitrypsin deficiency, systemic immune-driven disorders).
16. The use of prescription or over-the-counter medications is subjected to protocol established restrictions (non-permitted medications).
17. Any surgical or medical condition which might significantly alter the absorption, distribution, metabolism, or excretion of drugs, or which may jeopardize the subject in case of participation in the study. The investigator must determine this in consideration of the subject's medical history and/or clinical or laboratory evidence of the following conditions, including but not limited to: inflammatory bowel disease; digestive tract ulcers; gastrointestinal or rectal bleeding; major gastrointestinal tract surgery such as gastrectomy or bowel resection; pancreatic injury or pancreatitis; liver disease or liver injury as indicated by abnormal liver function analytes such as SGOT (AST), SGPT (ALT), γ-GT, or alkaline phosphatase.
18. Subjects that are receiving, or have received within the past 5 years, specific immunotherapy.

### Study Design

A phase II, double-blind, randomized, parallel-group, placebo-controlled multi-center study to investigate the effect of the adenosine A₁ receptor antagonist Compound (I) on forced expiratory volume in 1 second (FEV1) in patients with mild-to-moderate persistent atopic asthma.

The primary objective is to demonstrate an improvement in trough FEV1 upon a 15-day treatment with Compound (I) compared to placebo in mild-to-moderate asthmatics that, on study entry, are managed in GINA therapeutic steps 2-3 (except for the use of high dose inhaled corticosteroid -ICS-) or step 4 restricted to medium-dose ICS plus long-acting β2-agonist (LABA) bronchodilator and/or a leukotriene receptor antagonist (LTRA), as maintenance therapy. Secondary objectives include determinations of FEV1 area under the curve (AUC), evaluations on pre- and post- bronchodilator FEV1, and patient reported outcomes (PROs) including Asthma Control Questionnaire-7 (ACQ-7) and Standardized Asthma Quality of Life Questionnaire (AQLQ(S)).

The study comprises: (i) a minimum of 5-days screening period, during which the subject's clinical stability and overall eligibility for the study will be assessed; (ii) a weaning phase where a stepwise tapering of the asthma medication will be done upon 7-day periods; (iii) the randomized, parallel-arm treatment period; and (iv) an end-of-study follow-up visit. The asthma medication weaning period comprises three possible visit pathways in order to adjust for each subject's asthma therapy on study entry. The study comprises a primary analysis population of 58 stable asthmatic subjects managed as described for the objectives, who meet all inclusion criteria and no exclusion criteria and complete a full, valid data set for the primary variable. Figure 1 shows the trial design.

Data analysis. The primary efficacy variable and secondary outcome distributions will be analyzed by treatment using a repeated measures model where appropriate. Other analyses will comprise data sets generated from baseline characteristics and safety assessments, pharmacokinetics, and discretionary analyses to evaluate the influence of baseline and clinical covariates on the primary variable. Factor-tailored sub-analyses may be performed as per an adaptive data analysis plan.

### Results

Of the 107 subjects who were screened, 63 (58.9%) met eligibility criteria and were randomized. Of the 63 patients who were randomized, 63 (100%) completed the study (32 and 31 in the Compound (I) and placebo groups, respectively). There were not adverse events for discontinuation. The safety population included 63 (placebo: n=31 and Compound (I): n=32). Results are based on the per protocol population (placebo: n= 27 and Compound (I): n= 31).

Patient demographic and baseline characteristics of Safety Population are summarized in Table 1.

**Table 1**

| | **Compound (I) 10 mg N= 32** | **Placebo N= 31** | **Total N= 63** |
|---|---|---|---|
| Age | | | |
| Mean (SD) | 35.4 (11.7) | 34.6 (10.0) | |
| Median | 35.0 | 33.0 | |

| Gender | | | |
|---|---|---|---|
| Female n (%) | 19 (59.4) | 17 (54.8) | 36 |
| Male n (%) | 13 (40.6) | 14 (45.2) | 27 |
| BMI (kg/m²) Mean (SD) | 25.8 (6.4) | 27.1 (7.0) | |

| Race | | | |
|---|---|---|---|
| White n (%) | 31 (96.9) | 29 (93.5) | 60 |
| Black n (%) | 1 (3.1) | 0 (0.0) | 1 |
| Asian n (%) | 0 (0.0) | 1 (3.2) | 1 |
| remaining | 0 (0.0) | 1 (3.2) | 1 |

### - Change from baseline in blood eosinophils count

Overall change from baseline in blood eosinophil count (10⁹/L) after 15 days of treatment showed treatment differences (reduction in blood eosinophil count) for the Compound (I) group compared with placebo. Table 2 and Figure 2.

**Table 2**

| **Variable (unit)** | **Statistic** | **Compound (I) 10 mg N=31** | **Placebo N=27** | **p-value** |
|---|---|---|---|---|
| Baseline Eosinophilic Count (cel/ µL) On visit V5 | Mean | 392.9 | 382.6 | |
| | SD | 203.5 | 200.5 | |
| | Mean (95% CI) | 250.0 (467.5) | 210.0 (461.9) | |
| | Median | 370.0 | 380.0 | |
| | Min, max | 100.0, 980.0 | 90.0, 1010.0 | |
| | | **N=30** | **N=27** | |
| Eosinophilic Count (cel/ µL) on Visit V7 (day 15) | Mean | 218 | 431.9 | |
| | SD | 250.9 | 206.5 | |
| | Mean (95% CI) | 60.1 (311.7) | 260.0 (513.6) | |
| | Median | 150.0 | 400.0 | |
| | Min, max | 0.0, 1250.0 | 60.0, 870.0 | |
| | | | | |
| Absolute Change from Baseline | Mean | -184.7 | 49.3 | |
| | SD | 204.5 | 136.5 | |
| | Mean (95% CI) | -261.0 (-108.3) | -4.7 (103.3) | |
| | Median | -195.0 | 30.0 | |
| | Min, max | -700.0, 270.0 | -220.0, 350.0 | |
| Mean difference vs Placebo (90% CI) | - | -233.9 (-Inf, -174.6) | - | |
| Relative Change from Baseline | Mean | -46.8 | 21.2 | |
| | SD | 44.8 | 48.5 | |
| | Mean (95% CI) | -63.5, -30.0 | 2.0, 40.4 | |
| | Median | -64.9 | 5.5 | |
| | Min, max | -100.0, 66.7 | | |
| | Mean difference vs Placebo (90% CI) | -68.0 (-Inf, -51.95) | | |
| | | | | <0.0001 |

As seen from the table above, the decrease eosinophilic count in the subjects treated with Compound (I) was statistically and clinically significant and associated with improved clinical outcomes during the trial.

The administration of Compound (I) decreases peripheral blood eosinophils level between 5% - 30% from baseline.

### - Analysis of peripheral blood eosinophils level by patient subgroup.

Table 3. Summary of values for categorized Eosinophils (cel/ µL) at visit V1 (Per Protocol Population).

**Table 3**

| | **Compound (I) 10 mg N= 31** | **Placebo N= 27** | **Total N= 58** |
|---|---|---|---|
| **Eosinophils (cel/µL) at visit V1** | | | |
| Low n (%) | 13 (41.9) | 16 (59.3) | 29 (50) |
| High n (%) | 18 (58.1) | 11 (40.7) | 29 (50) |

| | | | |
|---|---|---|---|
| Low <=300 cel/µL; High >300 cel/µL. | | | |

Table 4. Mixed Model using Primary Efficacy endpoint (FEV1 in L) in the different Eosinophils (cel/ µL) category at visit V1 (Per Protocol Population).

**Table 4**

| **LS means** | **Estimate (SE)** | **Estimate (CI 90)** | **p value** |
|---|---|---|---|
| Study arm* Eosinophils category Compound (I)* High | 3.06 (0.11) | 2.89, 3.24 | < 0.0001 |
| Study arm* Eosinophils category Compound (I)* Low | 3.04 (0.12) | 2.83, 3.24 | < 0.0001 |
| Study arm* Eosinophils category Placebo* High | 2.69 (0.14) | 2.45, 2.92 | < 0.0001 |
| Study arm* Eosinophils category Placebo* Low | 2.99 (0.11) | 2.80, 3.19 | < 0.0001 |

| **LS means differences in study arm** | | | |
|---|---|---|---|
| Study arm Compound (I) vs Placebo | 0.21 (0.11) | 0.05, Inf | 0.0596 |

| **LS means differences** | | | |
|---|---|---|---|
| Study Arm* Eosinophils category High* (COMPOUND (I) - Placebo) | 0.38 (0.15) | 0.12, 0-63 | 0.0164 |
| Study Arm* Eosinophils category Low* (COMPOUND (I) - Placebo) | 0.04 (0.15) | -0.20, 0.29 | 0.7778 |

| | | | |
|---|---|---|---|
| LS: Least-squares. | | | |

As seen from Table 4, those patients with high level of eosinophils at V1 (>=300 cel/µL) experimented a statistically significant improvement in trough FEV1 (0.38 L; p=0.0164) compared to the corresponding placebo. On the contrary, in those patients with low level of eosinophils at V1 (<300 cel/µL) the treatment lead to a non-statistically significant improvement of 0.04 L (p=0.7778) in trough FEV1 compared to the corresponding placebo.

### - Change from baseline in trough FEV1

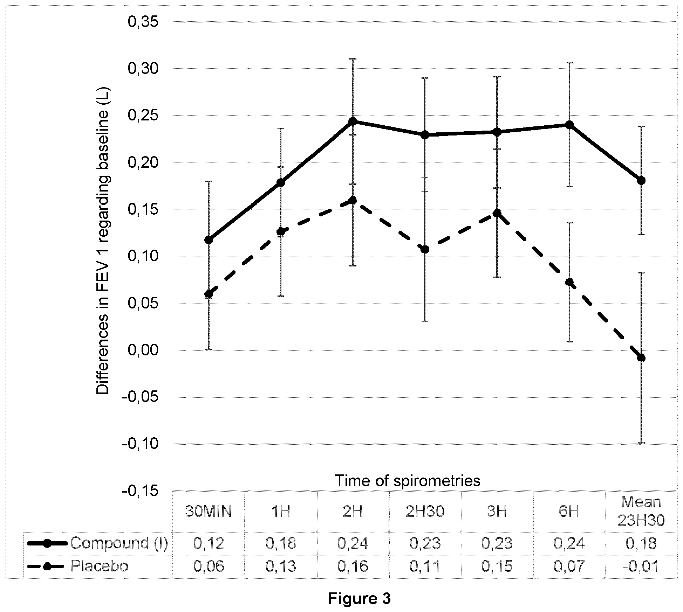

The analysis of the primary efficacy variable, overall change from baseline in trough FEV1 over 15-days of treatment showed significant improvement (increase) for patients in Compound (I) group compared with placebo (Table 5 and Figure 3). A mixed model with FEV1 measurements on day 16 (23h 15min and 23h 45min post-dose), containing treatment arm as a fixed effect, site as random effect with baseline FEV1 on visit V5 as a covariate was fitted. The treatment effect was larger for Compound (I) group compared to placebo, increasing the FEV1 on 180 mL (p= 0.0816).

**Table 5**

| **Variable (unit)** | **Statistic** | **Compound (I) 10 mg N=31** | **Placebo N=27** | **p-value** |
|---|---|---|---|---|
| Baseline FEV1 (liters) V5 | Mean | 2.7 | 2.9 | |
| | SD | 0.7 | 0.8 | |
| | 95% CI | 2.5, 3.0 | 2.6, 3.2 | |
| | Median | 2.8 | 2.9 | |
| | Min, max | 1.3, 4.3 | 1.7, 4.2 | |
| Trough FEV1 day 16 (+4-day leeway) | LS mean | 3.1 | 2.9 | |
| | SE of LS mean | 0.09 | 0.10 | |
| | 90% CI | 2.87, 3.25 | 2.66, 3.10 | |
| | LS mean difference (SE) vs. Placebo | 0.18 | - | |
| | SE of difference | 0.11 | - | |
| | 90% CI | 0.02, Inf | - | |
| | | - | | 0.0816 |

| | | | | |
|---|---|---|---|---|
| FEV1: forced expiratory volume in 1 second; SD: standard deviation; SE: standard error; min: minimum; max: maximum; LS: least squares; CI: confidence interval. Trough FEV1 on day 16 (+4-day leeway), defined as the average of the FEV1 measurements taken at 23h15min and 23h45min post-dose. | | | | |

Specifically, the administration of Compound (I) increases trough FEV1 between 110-200 mL.

### - Change from baseline in FEV1 AUC₃₀ₘᵢₙ₋₂₃ₕ₃₀ₘᵢₙ post-dose through days 15-to-16

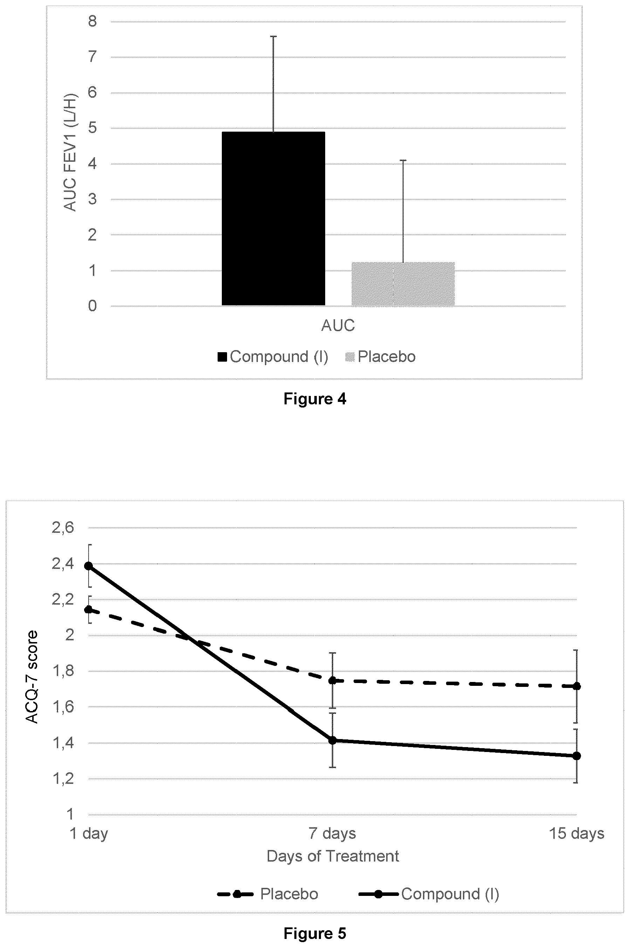

The analysis of the secondary efficacy variable, overall change from baseline in FEV1 AUC₃₀ₘᵢₙ₋₂₃ₕ₃₀ₘᵢₙ post-dose through days 15-to-16 after treatment showed significant improvement (increase) for patients in Compound (I) group compared with placebo (Table 6) and Figure 4.

**Table 6**

| **Variable (unit)** | **Statistic** | **Compound (I) 10 mg N=31** | **Placebo N=27** | **p-value** |
|---|---|---|---|---|
| FEV1 AUC_{30min- 23h30min} post-dose through days 15-to-16 | Mean | 4.9 | 1.2 | |
| | SD | 7.4 | 8.4 | |
| | 95% CI | 2.2, 7.6 | -2.1,4.5 | |
| | Median | 5.7 | 1.4 | |
| | Min, max | -9.1, 20.7 | -14.5, 26.6 | |
| | Mean difference vs Placebo (90% CI) | 3.6 (0.96) | - | |
| | | | | 0.0435 |

### - Change from baseline in Asthma Control Questionnaire-7 (ACQ-7)

The analysis overall mean change from baseline in ACQ-7 score over 15-days of treatment showed improvement (decrease) for patients in the Compound (I) group compared with placebo group (p= 0.0430). Table 7 and Figure 5. Similar trend was observed when comparing Visit and Study Arm* Visit as covariate.

**Table 7**

| **Variable** | | **p-value** | **Mean difference vs Placebo (90% CI)** | **Comments** |
|---|---|---|---|---|
| Mixed model with ACQ-7 overall score measurements on days 8 (+/- 1-day leeway) and 15 (+/- 4-days leeway) containing treatment arm and visit as fixed effect, site as random effect with baseline ACQ-7 overall score on visit V5 as a covariate | Effect: Treatment | 0.0430 | -0.28 | Significant |

### - Safety Profile

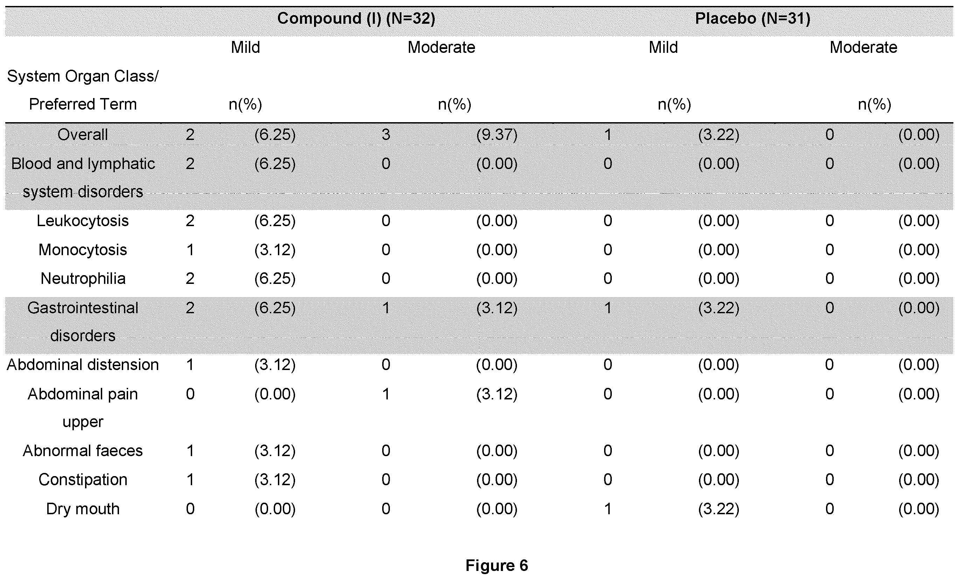

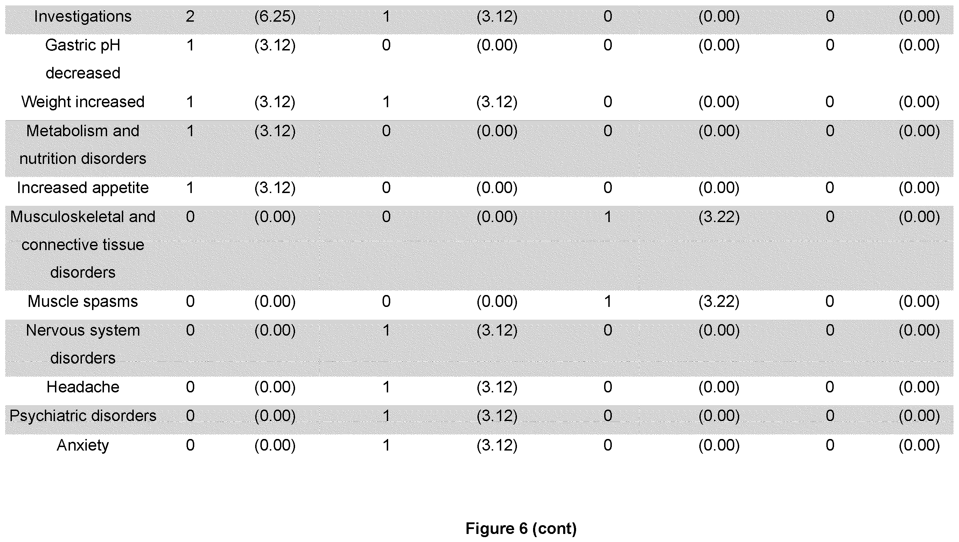

In connection with data above, Compound (I) shows a very good safety profile in allergic/atopic asthmatics. The data highlight the potential of the adenosine A₁ receptor antagonist Compound (I) as a promising oral asthma therapy. See Figure 6. The safety set included all randomized patients who received at least one dose of any study drug.

Overall, 63 patients completed the study (safety population) and 58 were evaluated in the per protocol (pp) population, which excluded non-compliance subject. There were significant differences between Compound (I) and placebo for peripheral eosinophils count, trough forced expiratory volume in 1s (FEV1) and Asthma control questionnaire-7 (ACQ-7) in the pp population. Patients treated with Compound (I) had significant improvement compared with placebo in trough FEV1, and a remarkable reduction of blood eosinophils produced upon a 15-days treatment.

Most adverse events (AEs) were mild/moderate in both groups, with no serious AEs reported. The Compound (I) showed a very good safety profile in allergic/atopic asthmatics.

## Claims

1. Compound of formula (I): 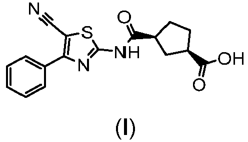 for use in the treatment of airway diseases in a subject having, before said treatment, a level of eosinophils in peripheral blood equal to or greater than 300 cel/µL comprising administering to said subject a therapeutically effective amount of said compound.

2. Compound according to claim 1 wherein eosinophilic airway disease is selected from allergic asthma, chronic obstructive pulmonary disease (COPD), idiopathic pulmonary fibrosis (IPF), obstructive sleep apnea and allergic rhinitis.

3. Compound according to claim 2 wherein the allergic asthma is selected from mild, moderate and severe asthma.

4. Compound according to any one claims 1 to 3 wherein the compound is administered by oral route.

5. Compound according to any one claims 1 to 4 wherein the compound is administered once or twice a day.

6. Compound according to claim 5 wherein the compound is administered once a day.

7. Compound according to any one claims 1 to 6 wherein the compound is administered at a dose between 5 - 40 mg.

8. Compound according to claim 7 wherein the compound is administered at a dose between 5 - 20 mg.

9. Compound according to any one claims 1 to 8 wherein the administration of Compound (I) decreases peripheral blood eosinophils level between 5%-30% from baseline.

10. Compound according to any one claims 1 to 9 wherein the administration of Compound (I) increases trough FEV1 between 110- 200 mL.

11. Compound for use according to claim 1 wherein compound (I) is present in a combination product comprising a compound of formula (I) or a pharmaceutically acceptable salt or co-crystal thereof, and one or more agent selected from the group consisting of corticosteroids, such as budesonide, fluticasone, beclomethasone, mometasone, bronchodilators such as salmetherol and formoterol, and biologic products selected from Dupilumab, Reslizumab, Mepolizumab, Imatinib, Lebrikizumab, AK002, Benralizumab, Tralokinumab, and anti-fibrotic drugs such as Pirfenidone and Nintedanib.
